# FASCICULE DE BREVET EUROPEEN

(11) **EP 0 768 199 B1**
(45) Date de publication et mention de la délivrance du brevet: **08.03.2000**
(21) Numéro de dépôt: 96440085.7
(22) Date de dépôt: 11.10.1996
(51) Int. Cl.: B60H 3/00, A61L 9/12

(54) **Dispositif desodorisant adaptable sur un aerateur de voiture**
Luftverbesserer zum Anbringen an einer Fahrzeug-Belüftungsdüse
Air freshener for fitting to a vehicle air vent

(30) Priorité: 12.10.1995 FR 9512191
(43) Date de publication de la demande: 16.04.1997
(73) Titulaire: Scheuer, Jean-Louis, 67320 Drulingen (FR); Felten, Marc, 67000 Strasbourg (FR)
(72) Inventeur: Scheuer, Jean-Louis, 67320 Drulingen (FR); Felten, Marc, 67000 Strasbourg (FR)
(74) Mandataire: Rhein, Alain

(56) Documents cités:
- EP-A- 0 328 702
- GB-A- 2 263 404
- US-A- 4 523 870

## Description

La présente invention a pour objet un dispositif désodorisant adaptable sur un aérateur de véhicule automobile ou autre système de ventilation.

On connaît déjà des dispositifs désodorisants adaptables sur les aérateurs des véhicules automobiles, ils comportent généralement un boîtier renfermant un matériau absorbant imprégné d'un produit parfumé, comportant des orifices que l'on peut éventuellement plus ou moins ouvrir pour permettre la diffusion du parfum, et présentant à sa face arrière des pattes sensiblement parallèles, plus ou moins souples destinées à permettre le pincement des ailettes d'un aérateur, l'ensemble venant de moulage d'une matière plastique.

Le principal inconvénient de ces dispositifs est de pas être adaptables à tous les aérateurs du marché, en effet l'écartement des pattes est fixe, et si l'accrochage est réalisable en jouant sur l'élasticité des pattes lorsque leur écartement n'est pas assez important par rapport à l'épaisseur des ailettes, il en est autrement lorsque ledit écartement est trop important.

La présente invention a pour but de remédier à cet inconvénient en proposant un dispositif désodorisant adaptable sur tous les aérateurs existants, tout en étant d'un coût de fabrication moindre que celui des dispositifs existants.

Le dispositif selon l'invention se caractérise en ce qu'il comporte d'une part un étui plat renfermant un matériau absorbant imprégné d'un produit parfumé, percé d'orifices permettant la diffusion du parfum, et d'autre part, faisant saillie de sa face arrière, un bloc de matière aisément compressible destiné à être introduit entre les ailettes d'un aérateur.

Selon une caractéristique additionnelle du dispositif selon l'invention, l'extrémité libre du bloc de matériau compressible comporte au moins une fente longitudinale, perpendiculaire à la face de l'étui à laquelle ledit bloc est solidarisé.

Les avantages et les caractéristiques de la présente invention ressortiront plus clairement de la description qui suit et qui se rapporte au dessin annexé, lequel en représente un mode de réalisation non limitatif.

Dans le dessin annexé :

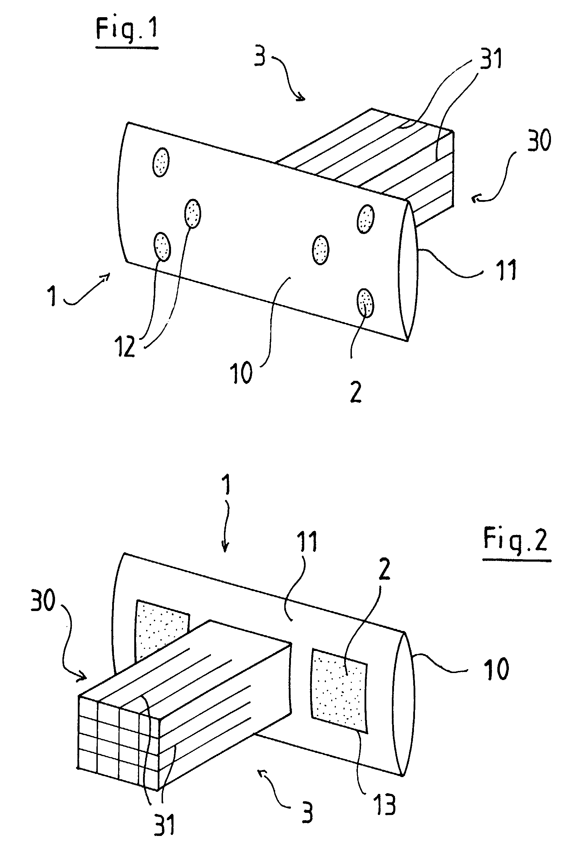

- la figure 1 représente une vue en perspective de face d'un dispositif selon l'invention.
- la figure 2 représente une vue en perspective de dos du même dispositif.

Si on se réfère à ces figures, on peut voir qu'un dispositif selon l'invention comporte un étui rectangulaire plat 1 renfermant un matériau absorbant 2 imbibé d'un produit parfumé, et dont les faces avant 10 et arrière 11 comportent chacune des orifices, respectivement 12 et 13, permettant la diffusion du parfum.

L'étui 1 est réalisé en un matériau semi-rigide du type matière plastique ou carton.

De la face arrière 11 fait saillie perpendiculairement un bloc parallélépipèdique 3 réalisé en un matériau souple compressible du type mousse plastique.

La compressibilité du bloc 3 permet son introduction entre deux ailettes d'un aérateur, son expansion au-delà des ailettes permettant la solidarisation, laquelle est largement suffisante du fait du faible poids de l'étui 1.

L'extrémité libre 30 du bloc 3 comporte des fentes longitudinales 31 permettant de fixer ce dernier sur l'aérateur en engageant l'une des ailettes dans l'une des fentes 31, la solidarisation étant réalisé par pincement.

L'utilisation de ce mode de solidarisation est plus particulièrement réservé aux aérateurs dont les ailettes sont peu épaisses et/ou très largement espacées.

Le dispositif selon l'invention pourra ainsi être aisément solidarisé à la grille de protection d'un ventilateur qui est généralement constituée d'un entrelacement de fils métalliques.

## Revendications

1. Dispositif désodorisant adaptable sur un aérateur de véhicule automobile, caractérisé en ce qu'il comporte d'une part un étui plat (1) renfermant un matériau absorbant (2) imprégné d'un produit parfumé, percé d'orifices (12, 13) permettant la diffusion du parfum, et d'autre part, faisant saillie de sa face arrière (11), un bloc (3) de matière aisément compressible destiné à être introduit entre les ailettes d'un aérateur.

2. Dispositif selon la revendication 1 caractérisé en ce que l'extrémité libre (30) du bloc (3) de matériau compressible comporte au moins une fente longitudinale (31), perpendiculaire à la face (11) de l'étui (1) à laquelle ledit bloc (3) est solidarisé.

3. Dispositif selon la revendication 1 ou la revendication 2 caractérisé en ce que le bloc (3) de matériau compressible est réalisé en une mousse plastique.

## Patentansprüche

1. Desodorisierende Vorrichtung, die auf den Belüfter eines Fahrzeuges angepaßt werden kann, dadurch gekennzeichnet, daß sie einerseits ein flaches Gehäuse (1) umfaßt, das ein mit einem parfümierten Produkt imprägniertes, absorbierendes Material (2) umschließt und mit Löchern (12, 13) durchbohrt ist, die die Abgabe des Parfums ermöglichen, und andererseits mit seiner hinteren Seite (11) einen Vorsprung mit einem Block (3) aus leicht komprimierbarem Material bildet, das dazu geeignet ist, zwischen den Flügeln eines Belüfters eingeführt zu werden.

2. Vorrichtung gemäß Anspruch 1, dadurch gekennzeichnet, daß das freie Ende (30) des Blocks (3) des komprimierbaren Materials wenigstens einen senkrecht zur Seite (11) des Gehäuses (1) verlaufenden länglichen Schlitz (31) beinhaltet, mit dem der besagte Block (3) fest verbunden ist.

3. Vorrichtung gemäß Anspruch 1 oder 2, dadurch gekennzeichnet, daß der Block (3) des komprimierbaren Materials aus einem Plastikschaumstoff realisiert wird.

## Claims

1. Deodorising device able to be adapted to a motor vehicle ventilator, characterised in that it firstly comprises a flat case (1) containing an absorbent material (2) impregnated with a scented product and pierced with openings (12, 13) enabling the perfume to be diffused, and secondly projecting from its rear face (11) a block (3) made of an easily compressible material intended to be introduced between the vanes of a ventilator.

2. Device according to claim 1, characterised in that the free end (30) of the compressible block (3) comprises at least one longitudinal aperture (31) perpendicular to the face (11) of the case (1) with which said block (3) is rendered integral.

3. Device according to claim 1 or 2, characterised in that the compressible block (3) is constituted by a plastic foam.
